# EUROPEAN PATENT SPECIFICATION

(11) **EP 4 079 548 B1**
(45) Date of publication and mention of the grant of the patent: **15.04.2026**
(21) Application number: 22158468.3
(22) Date of filing: 24.02.2022
(51) Int. Cl.: B60H 1/00, G01N 27/00, A61L 9/16, B60H 3/06

(54) **IN-CAR AIR POLLUTION PREVENTION SYSTEM**
SYSTEM ZUR VERHINDERUNG VON LUFTVERSCHMUTZUNG IM AUTO
SYSTÈME EMBARQUÉ DE PRÉVENTION CONTRE LA POLLUTION DE L'AIR

(30) Priority: 23.04.2021 TW 110114805
(43) Date of publication of application: 26.10.2022
(73) Proprietor: Microjet Technology Co., Ltd., Hsinchu (TW)
(72) Inventor: Mou, Hao-Jan, Hsinchu (TW); Wu, Chin-Chuan, Hsinchu (TW); Lin, Ching-Sung, Hsinchu (TW); Han, Yung-Lung, Hsinchu (TW); Huang, Chi-Feng, Hsinchu (TW); Tsai, Chang-Yen, Hsinchu (TW); Lee, Wei-Ming, Hsinchu (TW)
(74) Representative: Uexküll & Stolberg

(56) References cited:
- EP-A1- 2 106 940
- EP-A1- 3 632 723
- WO-A1-2018/156136
- FR-A1- 3 055 585
- FR-A1- 3 062 604
- GB-A- 2 548 621
- GB-A- 2 563 008
- US-A1- 2018 319 256

## Description

### FIELD OF THE INVENTION

The present disclosure relates to an air pollution filtration executed in a car, and more particularly to an in-car air pollution prevention system.

### BACKGROUND OF THE INVENTION

With the growths of global population and the rapid development in industry, the air quality is deteriorating gradually. It is not only harmful to human health but also life-threatening in severe cases for people to expose in the harmful air pollution gases for a long time.

There are many pollutants in the air, such as carbon dioxide, carbon monoxide, formaldehyde, bacteria, fungi, volatile organic compound (VOC), particulate matter 2.5 (PM_{2.5}) or ozone, etc. which may be seriously harmful to the human body as the concentration of pollutants increases. In the case of PM_{2.5}, such fine particles might penetrate through the alveoli, enter the blood vessels, and circulate throughout the body along with the blood circulation. As a result, they not only might be harmful to the respiratory tract, but also might lead to cardiovascular disease and/or increases the risk of cancer.

Nowadays, the prevalence of epidemic diseases, such as influenza and pneumonia, not only threatens people's health, but also restricts people's social activities, and the willingness to take public transportation has also decreased. As a result, driving by themselves has become the first choice of transportation when people need to go out. Therefore, how to make sure that the air in the vehicle is clean and safe for people to breath at all times during driving by people becomes an important research and development topic of the present disclosure. Document US 2018/319256 A1 represents an in-car air pollution prevention system for exchanging and filtering air pollution source in an interior space of a car.

### SUMMARY OF THE INVENTION

An object of the present disclosure is to provide an in-car air pollution prevention system for executing an air pollution filtration in an interior space of a car, so that the air pollution source in the interior space of the car can be filtered rapidly, so as to provide clean, safe and breathable air.

The invention is set out in the appended set of claims.

### BRIEF DESCRIPTION OF THE DRAWINGS

The above contents of the present disclosure will become more readily apparent to those ordinarily skilled in the art after reviewing the following detailed description and accompanying drawings, in which:
FIG. 1A is an exemplary schematic diagram illustrating an air detection module and an air conditioning device installed in a car according to the embodiment of the present disclosure;
FIG. 1B is an exemplary schematic diagram illustrating the air conditioning device installed in the car according to the embodiment of the present disclosure;
FIG. 2A is a schematic cross-sectional view illustrating the air conditioning device and the filtering and purification component according to the embodiment of the present disclosure;
FIG. 2B is an exemplary schematic diagram illustrating the air detection modules, the air conditioning device and the filtering and purification components installed in the car according to the embodiment of the present disclosure;
FIG. 2C is a schematic cross-sectional view illustrating the air outlet of the air conditioning device and the filtering and purification component according to the embodiment of the present disclosure;
FIG. 2D is a schematic cross-sectional view illustrating the air inlet of the air conditioning device and the filtering and purification component according to the embodiment of the present disclosure;
FIG. 2E is a schematic cross-sectional view illustrating the filtering and purification component according to the embodiment of the present disclosure;
FIG. 3 is a schematic perspective view illustrating the air detection module according to the embodiment of the present disclosure;
FIG. 4A is a schematic front perspective view illustrating the air detection main part according to the embodiment of the present disclosure;
FIG. 4B is a schematic rear perspective view illustrating the air detection main part according to the embodiment of the present disclosure;
FIG. 4C is an exploded view illustrating the air detection main part according to the embodiment of the present disclosure;
FIG. 5A is a schematic front perspective view illustrating the base according to the embodiment of the present disclosure;
FIG. 5B is a schematic rear perspective view illustrating the base according to the embodiment of the present disclosure;
FIG. 6 is a schematic view illustrating the laser component combined within the base according to the embodiment of the present disclosure;
FIG. 7A is a schematic exploded view illustrating the combination of the piezoelectric actuator and the base according to the embodiment of the present disclosure;
FIG. 7B is a schematic perspective view illustrating the combination of the piezoelectric actuator and the base according to the embodiment of the present disclosure;
FIG. 8A is a schematic exploded front view illustrating the piezoelectric actuator according to the embodiment of the present disclosure;
FIG. 8B is a schematic exploded rear view illustrating the piezoelectric actuator according to the embodiment of the present disclosure;
FIG. 9A is a schematic cross-sectional view illustrating the piezoelectric actuator according to the embodiment of the present disclosure;
FIG. 9B is a schematic cross-sectional view illustrating a first operation step of the piezoelectric actuator according to the embodiment of the present disclosure;
FIG. 9C is a schematic cross-sectional view illustrating a second operation step of the piezoelectric actuator according to the embodiment of the present disclosure;
FIG. 10A is a schematic cross-sectional view illustrating the air of the air detection main part entering through the inlet opening of the outer cover according to the embodiment of the present disclosure;
FIG. 10B is a schematic cross-sectional view illustrating the light beam emitted by the laser component emitting a light beam to pass through the transparent window and be irradiated into the air-inlet groove according to the embodiment of the present disclosure;
FIG. 10C is a schematic cross-sectional view illustrating the air in the air-outlet groove being pushed to flow through the air outlet and the outlet opening and discharged out according to the embodiment of the present disclosure;
FIG. 11 is a schematic diagram illustrating a telecommunication connecting configuration of the in-car air detection module, the out-car air detection module and the control drive unit according to the embodiment of the present disclosure; and
FIG. 12 is a block diagram illustrating a telecommunication connecting configuration of the in-car air detection module, the out-car detection module, the controlling circuit board and the purification and filtration device according to the embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this invention are presented herein for purpose of illustration and description only. It is not intended to be exhaustive or to be limited to the precise form disclosed.

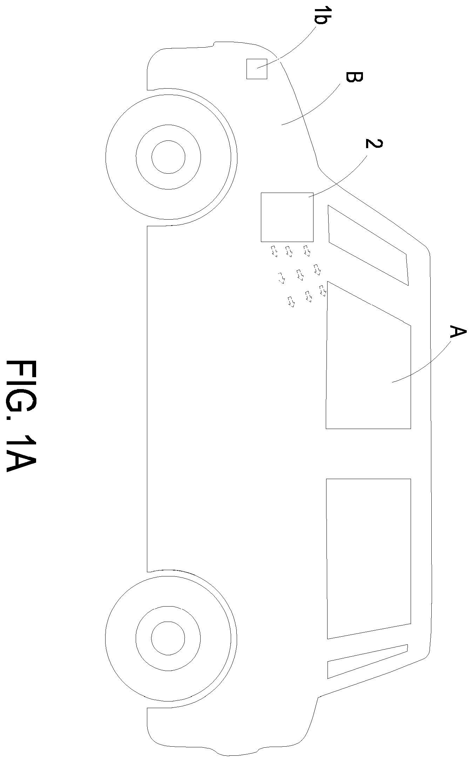

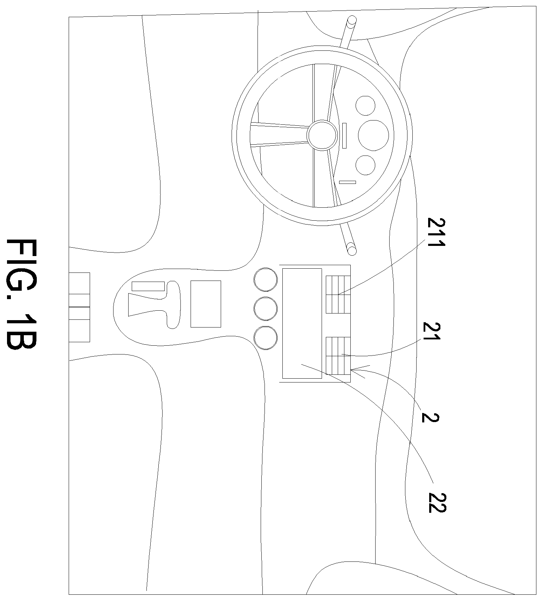

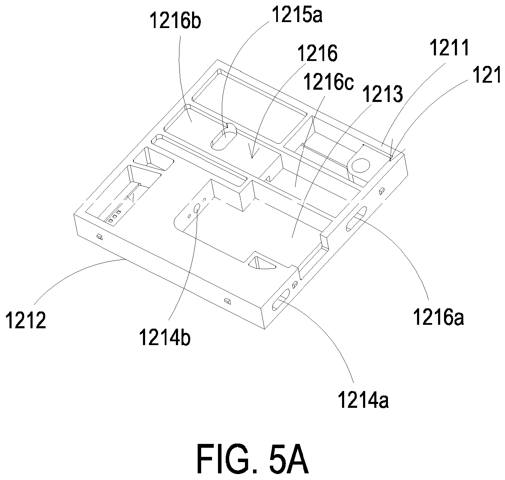

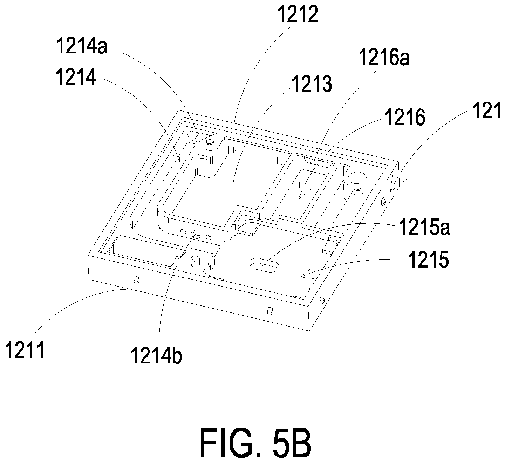

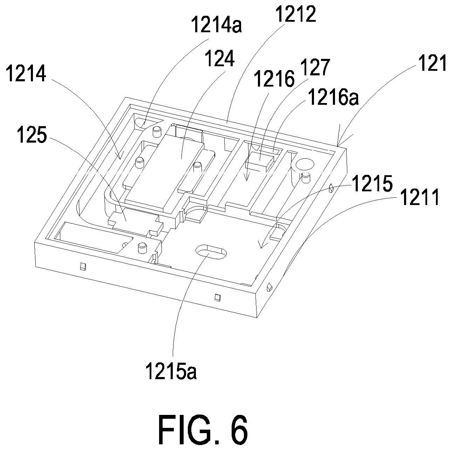

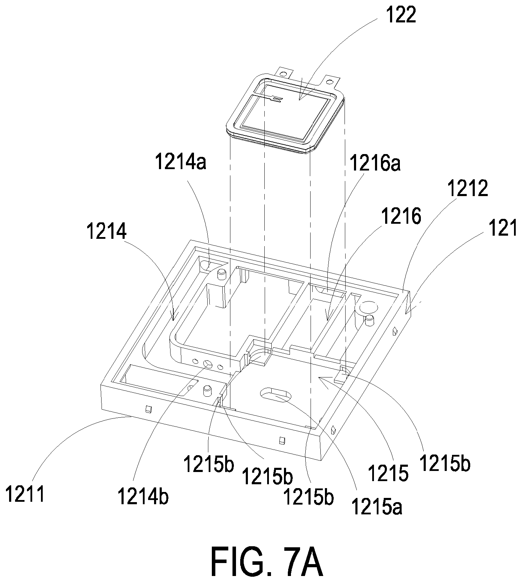

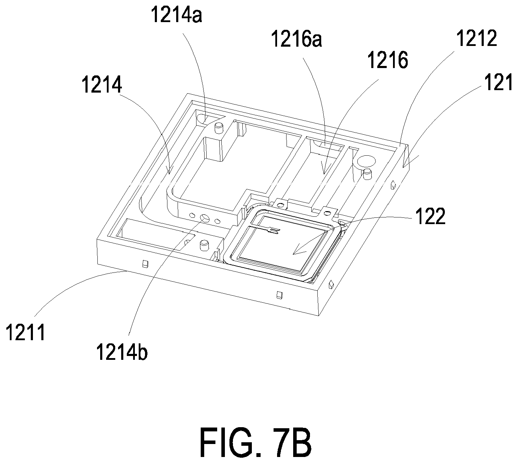

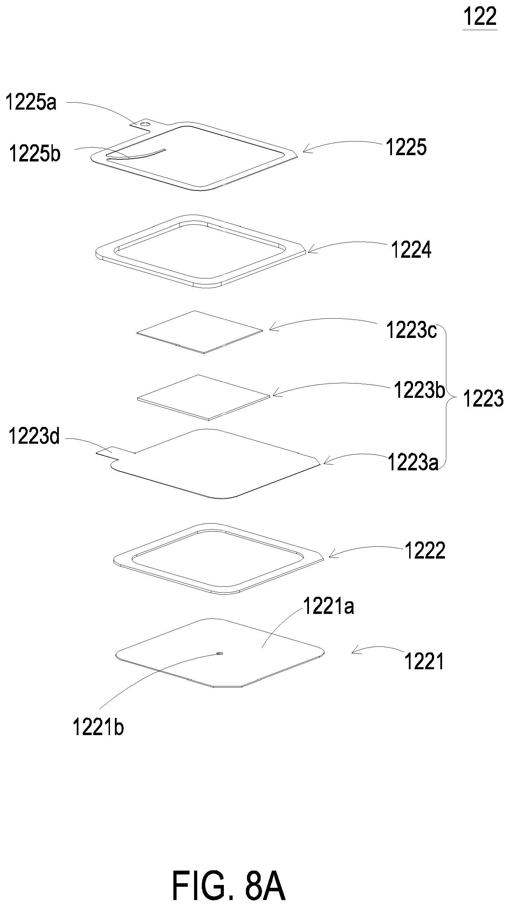

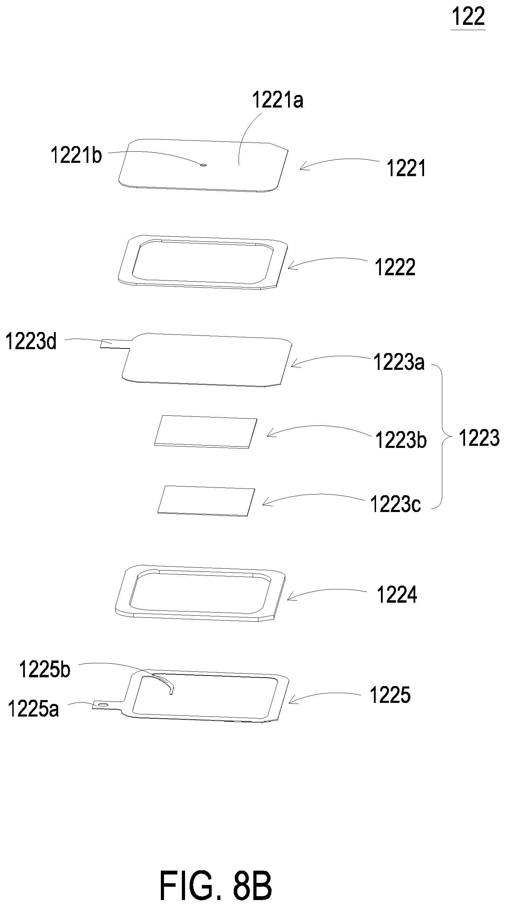

Please refer to FIGS. 1 to 12. The present disclosure provides an in-car air pollution prevention system for exchanging and filtering air pollution source in an interior space A of a car, so that the air pollution source in the interior space A of the car is filtered rapidly, so as to provide clean, safe and breathable air. The in-car air pollution prevention system includes a plurality of air detection modules, an air conditioning device 2 and a plurality of filtering and purification components D.

In the embodiment, the plurality of air detection modules includes a plurality of in-car air detection modules 1a and a plurality of out-car air detection modules 1b. The in-car air detection module 1a is disposed at the position of an external-air inlet 213 in the interior space A of the car for detecting an air pollution source in the interior space A of the car and transmitting an in-car air detection datum. Preferably but not exclusively, in the embodiment, the in-car air detection module 1a is a mobile detection device. That is, the in-car air detection module 1a may be a wearable device, such as a watch or a bracelet, which is directly worn on the human body (not shown). When people get into the interior space A of the car, the in-car air detection module 1b can detect the air pollution source in the interior space A of the car immediately in real-time at any time. In the embodiment, the out-car air detection module 1b is disposed in an exterior space B outside the car for detecting the external air in the exterior space B outside the car and transmitting an out-car air detection datum.

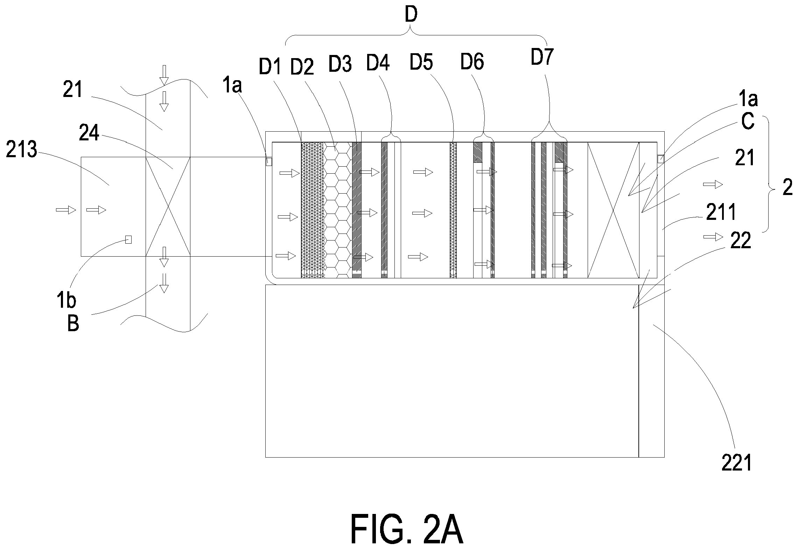

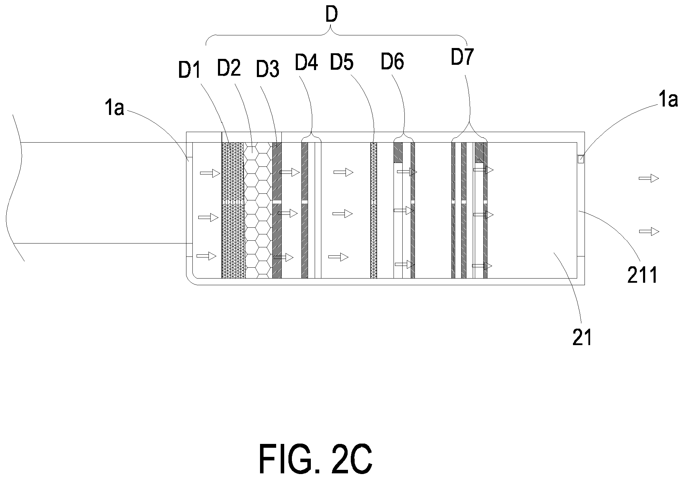

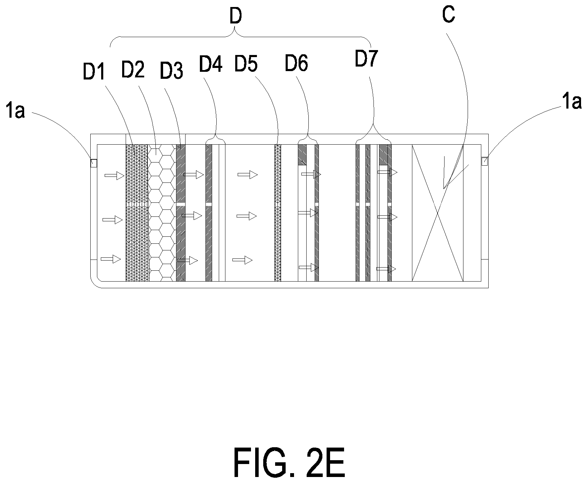

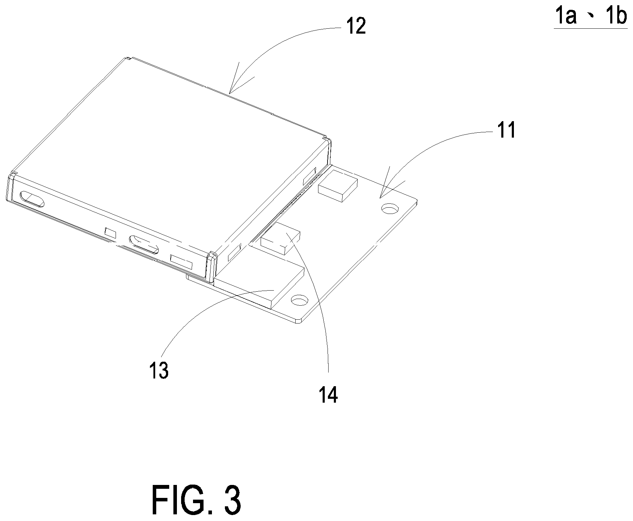

In the embodiment, the air conditioning device 2 controls the introduction or not introduction of the external air in an exterior space B outside the car into the interior space A of the car. In addition, the air conditioning device 2 includes a ventilation channel 21, a control drive unit 22 and an intake-control element 24. The control drive unit 22 includes a touch screen 221 for setting up the control instructions of the air conditioning device 2 by touching the touch screen 221 and displaying the in-car air detection datum. As shown in FIGS. 2A to 2D, the ventilation channel 21 includes at least one air outlet 211, at least one air inlet 212 and an external-air inlet 213, and an air guider C is disposed therein. Preferably but not exclusively, the air guider C guides the air to be discharge from the at least one air outlet 211 to, and inhale through the external-air inlet 213 and the at least one air inlet 212. As shown in FIG. 2C and FIG. 3D, the at least one air outlet 211 is in fluid communication with the ventilation channel 21 shown in FIG. 2A. In that, after the air is discharged out through the air outlet 211, the discharged air flows through the ventilation channel 21 and transported to the exterior space B outside the car.

In the embodiment, the filtering and purification components D are disposed at the positions of the air outlet 211 and the air inlet 212 of the ventilation channel 21 for filtering and purifying the external air and the air pollution source. Moreover, two in-car air detection modules 1a are disposed at two sides of the filtering and purification component D, respectively. Based on the air detection data before and after filtering are detected by the in-car air detection modules 1a on both sides, the control drive unit 22 is allowed to receive and compare the out-car air detection datum outputted by the out-car air detection module 1b and the in-car air detection datum outputted by the in-car air detection module 1a, so that the filter and purification component D is driven to filter the external air and the air pollution source, so as to generate and introduce clean air into the interior space A of the car.

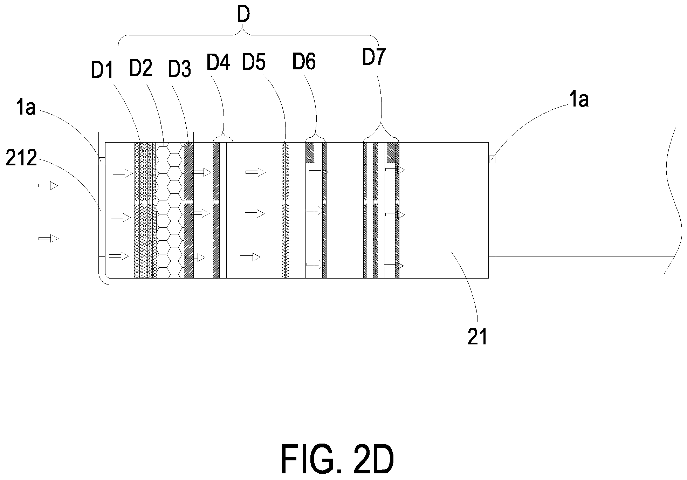

In the embodiment, the control drive unit 22 of the air conditioning device 2 controls the opening and closing of the air intake control member 24, and receives and compares the in-car air detection datum outputted from the in-car air detection module 1a and the out-car air detection datum outputted from the out-car air detection module 1b, so as to determine the opening and closing of the external-air inlet 213 and selectively control the introduction or not introduction of the external air in the exterior space B outside the car into the interior space A of the car, and control the air guider C of the air conditioning device 2 to be enabled immediately in a state of a monitoring mechanism. Please refer to FIG. 2A and FIG. 2D. The external-air inlet 213 and the air inlet 212 are controlled by the intake-control element 24 to open for introducing the external air in the exterior space B outside the car and the air pollution source in the interior space A of the car, respectively, into the air conditioning device 2. The external air and the air pollution source are filtered through the filtering and purification components D and exhausted into the exterior space B outside the car through the ventilation channel 21.

Preferably but not exclusively, in an embodiment, the control drive unit 22 of the air conditioning device 2 is configured to receive and compare the in-car air detection data outputted by at least three in-car air detection modules 1a under the calculation of artificial intelligence, so as to locate the position of the air pollution source in the interior space A of the car, and intelligently select and control the filtering and purification component D disposed in the position of the air inlet 212 adjacent to the air pollution source to accelerate the transportation for filtering.

Preferably but not exclusively, in another embodiment, the control drive unit 22 of the air conditioning device 2 is configured to receive and compare the in-car air detection data outputted by at least three in-car air detection modules 1a under the calculation of artificial intelligence, so as to locate the position of the air pollution source in the interior space A of the car, and intelligently select and control the air outlet 211 adjacent to the air pollution source to expel air in first priority, and direct the air pollution source toward the air inlet 212 adjacent thereto. At the same time, the control drive unit 22 of the air conditioning device 2 selects and controls the rest of the air outlets 211 to expel air under the calculation of artificial intelligence, so as to generate an airflow to direct the air pollution source toward the air inlet 212 adjacent to the air pollution source and to be inhaled for filtering rapidly.

In the embodiment, the state of the monitoring mechanism is enabled when at least one of the air detection data of the air pollution source detected by the plurality of air detection modules in the interior space A of the car is greater than a safe detection value. Preferably but not exclusively, the safe detection value comprises at least one selected from the group consisting of PM2.5 less than 35 µg/m³, carbon dioxide content less than 1000 ppm, total volatile organic compounds (TVOC) less than 0.56 ppm, formaldehyde content less than 0.08 ppm, the amount of bacteria less than 1500 CFU/m³, the amount of fungi less than 1000 CFU/m³, sulfur dioxide content less than 0.075 ppm, nitrogen dioxide content less than 0.1 ppm, carbon monoxide content less than 9 ppm, ozone content less than 0.06 ppm, lead content less than 0.15 µg/m³ and a combination thereof.

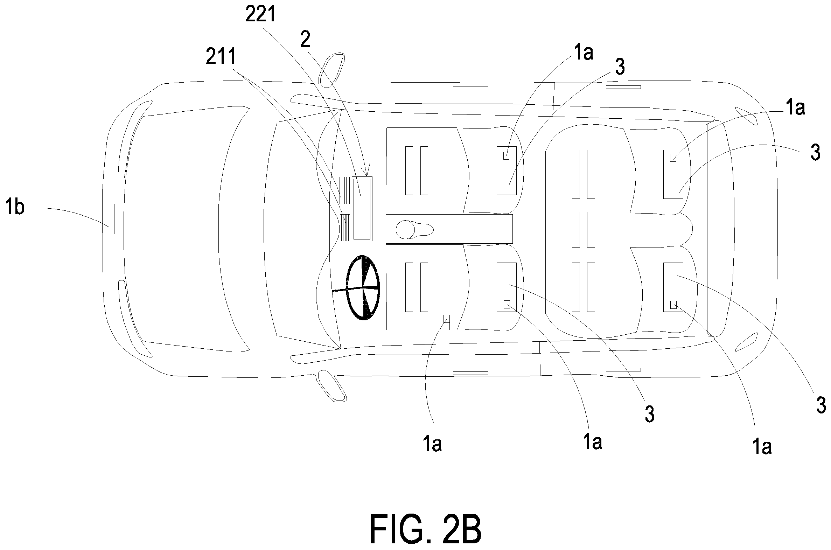

Please refer to FIGS. 2A and 2B. In another embodiment, the in-car air pollution prevention system of the present disclosure further includes at least one purification and filtration device 3. The purification and filtration device 3 includes the air guider C and the filtering and purification component D. The in-car air detection module 1a is combined with the purification and filtration device 3. In the embodiment, the in-car air detection module 1a transmits the in-car air detection datum to the control drive unit 22 of the air conditioning device 2, and the control drive unit 22 of the air conditioning device 2 receives and compares the in-car air detection datum, so as to intelligently select and control the enabling of purification and filtration device 3 adjacent to the air pollution source. In that, the air guider C is enabled, and the air pollution source in the interior space A of the car is guided and enters the filtering and purification component D of the purification and filtration device 3 for filtering and purifying. Preferably but not exclusively, in one embodiment, the purification and filtration device 3 is combined and embedded on a trim panel, a seat or a door pillar in the interior space A of the car.

Preferably but not exclusively, in an embodiment, the control drive unit 22 of the air conditioning device 2 is configured to receive and compare the in-car air detection data outputted by at least three in-car air detection modules 1a under the calculation of artificial intelligence, so as to locate the position of the air pollution source in the interior space A of the car, and intelligently select and control the enabling of the purification and filtration device 3 adjacent to the air pollution source, so the the air pollution source can be inhaled into the purification and filtration device 3 without diffusion to accelerate the filtration.

Preferably but not exclusively, in another embodiment, the control drive unit 22 of the air conditioning device 2 is configured to receive and compare the in-car air detection data outputted by at least three in-car air detection modules 1a under the calculation of artificial intelligence, so as to locate the position of the air pollution source in the interior space A of the car, and intelligently select and control the purification and filtration device 3 adjacent to the air pollution source to be enabled in first priority. At the same time, the control drive unit 22 of the air conditioning device 2 selects and controls the rest of the purification and filtration devices 3 to be enabled under the calculation of artificial intelligence, so that an airflow is generated to direct the air pollution source toward the purification and filtration device 3 adjacent to the air pollution source to be inhaled for filtering rapidly.

In the embodiment, at least one of the plurality of in-car air detection modules 1a is disposed at two sides of the plurality of purification and filtration devices 3, respectively. Based on the in-car air detection data before and after filtering are detected by the in-car air detection modules 1a on both sides, the control drive unit 22 can receive and compare the in-car air detection data outputted by the in-car air detection modules 1a located at the positions of the plurality of the purification and filtration devices 3 to ensure the air pollution source is filtered by the plurality of the purification and filtration devices 3, so as to generate another clean air introduced into the interior space A of the car.

In the embodiment, the state of the monitoring mechanism is enabled when at least one of the air detection data of the air pollution source detected by the plurality of air detection modules in the interior space A of the car is greater than a safe detection value. Preferably but not exclusively, the safe detection value comprises at least one selected from the group consisting of PM2.5 less than 35 µg/m³, carbon dioxide content less than 1000 ppm, total volatile organic compounds (TVOC) less than 0.56 ppm, formaldehyde content less than 0.08 ppm, the amount of bacteria less than 1500 CFU/m³, the amount of fungi less than 1000 CFU/m³, sulfur dioxide content less than 0.075 ppm, nitrogen dioxide content less than 0.1 ppm, carbon monoxide content less than 9 ppm, ozone content less than 0.06 ppm, lead content less than 0.15 µg/m³ and a combination thereof.

In the embodiment, the filtering and purification component D includes a combination of various implementations. Preferably but not exclusively, the filtering and purification component D can be a combination of an activated carbon D1 and a high efficiency particulate air (HEPA) filter screen D2; or a combination of an activated carbon D1, a high efficiency particulate air (HEPA) filter screen D2 and a zeolite screen D3. The activated carbon D1 is configured to filter and absorb the particulate matter 2.5 (PM_{2.5}), the zeolite screen D3 is configured to filter and absorb the volatile organic compounds (VOC), and the HEPA filter screen D2 is configured to absorb the chemical smoke, bacteria, dust particles and pollen contained in the air, so that the air pollution source introduced into the filtering and purification component D is filtered and purified to achieve the effect of filtering and purifying. In some embodiment, the HEPA filter screen D2 is coated with a cleansing factor containing chlorine dioxide layer, so as to inhibit viruses, bacteria and fungi contained in air introduced into the filtering and purification component D. Preferably but not exclusively, the HEPA filter screen D2 is coated with a cleansing factor containing chlorine dioxide layer, so as to inhibit viruses, bacteria, fungi, influenza A, influenza B, enterovirus and norovirus in the air pollution source introduced into the filtering and purification component D. The inhibition ratio is more than 99%, and it is helpful of reducing the cross-infection of viruses. In some embodiment, the HEPA filter screen D2 is coated with an herbal protective layer extracted from ginkgo and Japanese rhus chinensis to form an herbal protective anti-allergic filter, so as to resist allergy effectively and destroy a surface protein of influenza virus (H1N1) introduced by the filtering and purification component D and passing through the HEPA filter screen D2. In some embodiment, the HEPA filter screen D2 is coated with a silver ion, so as to inhibit viruses and bacteria contained in the air pollution source introduced by the filtering and purification component D.

In an embodiment, the filtering and purification component D includes the combination of an activated carbon D1, a high efficiency particulate air (HEPA) filter screen D2 and a zeolite screen D3, and a phot-catalyst unit D4. In that, the air pollution source is introduced into the filtering and purification component D and the light energy is converted into the chemical energy by the photo-catalyst unit D4, thereby decomposing harmful material in the air pollution source and disinfecting bacteria contained therein, so as to achieve the effects of filtering and purifying.

In an embodiment, the filtering and purification component D includes the combination of an activated carbon D1, a high efficiency particulate air (HEPA) filter screen D2 and a zeolite screen D3, and a photo-plasma unit D5. The photo-plasma unit D5 includes a nanometer irradiation tube. The air pollution source introduced by the filtering and purification component D is irradiated by the nanometer irradiation tube to decompose and purify volatile organic compounds contained in the air pollution source. When the air pollution source is introduced by the filtering and purification component D, the introduced air is irradiated by the nanometer irradiation tube, thereby oxygen molecules and water molecules contained in the air pollution are decomposed into high oxidizing photo-plasma, and generates an ion flow capable of destroying organic molecules. In that, volatile formaldehyde, volatile toluene and volatile organic compounds (VOC) contained in the air pollution are decomposed into water and carbon dioxide, so as to achieve the effects of filtering and purifying.

In an embodiment, the filtering and purification component D includes the combination of an activated carbon D1, a high efficiency particulate air (HEPA) filter screen D2 and a zeolite screen D3, and a negative ionizer D6. When the air pollution source introduced by the filtering and purification component D passes through a high voltage discharge, it makes the suspended particles in the air pollution source to carry with positive charge and adhered to the dust collecting plate carry with negative charges, so as to achieve the effects of filtering and purifying the air pollution source introduced.

In an embodiment, the filtering and purification component D includes the combination of an activated carbon D1, a high efficiency particulate air (HEPA) filter screen D2 and a zeolite screen D3, and a plasma ion unit D7. A high-voltage plasma column with plasma ion is formed by the plasma ion unit D7, so as to decompose viruses or bacteria contained in the air pollution source introduced by the filtering and purification component D. The oxygen molecules and water molecules contained in the air pollution source are decomposed into positive hydrogen ions (H⁺) and negative oxygen ions (O₂⁻) by the plasma ion. The substances attached with water around the ions are adhered on the surface of viruses and bacteria and converted into OH radicals with extremely strong oxidizing power, thereby removing hydrogen (H) from the protein on the surface of viruses and bacteria, and thus decomposing (oxidizing) the protein, so as to filter the introduced air pollution source and achieve the effects of filtering and purifying.

In an embodiment, the filtering and cleaning component D may merely include the HEPA filter screen D2. In an embodiment, the HEPA filter screen D2 is combined with any one of the phot-catalyst unit D4, the photo-plasma unit D5, the negative ionizer D6 and the plasma ion unit D7. In an embodiment, the HEPA filter screen D2 is combined with a combination of any two of the phot-catalyst unit D4, the photo-plasma unit D5, the negative ionizer D6 and the plasma ion unit D7. In an embodiment, the HEPA filter screen D2 is combined with a combination of any three of the phot-catalyst unit D4, the photo-plasma unit D5, the negative ionizer D6 and the plasma ion unit D7. Alternatively, the HEPA filter screen D2 is combined with the phot-catalyst unit D4, the photo-plasma unit D5, the negative ionizer D6 and the plasma ion unit D7.

Notably, the service life of the HEPA filter screen D2 is calculated based on the monitoring mechanism of the air detection data detected by the in-car air detection module 1a and the out-car air detection module 1a with reference to an accumulated operation time of the air guider C in the air conditioning device 2.

After understanding the method of notifying in-car air pollution of the present disclosure, the implementation devices for the method of notifying in-car air pollution of the present disclosure are described in detail as following.

Please refer to FIG. 3. In the embodiment, each of the in-car air detection module 1a and the out-car air detection module 1b includes a controlling circuit board 11, an air detection main part 12, a microprocessor 13 and a communicator 14. The air detection main part 12, the microprocessor 13 and the communicator 14 are integrally packaged on the controlling circuit board 11 and electrically connected to each other. In the embodiment, the microprocessor 13 and the communicator 14 are mounted on the controlling circuit board 11. The microprocessor 13 controls the driving signal of the air detection main part 12 for enabling the detection, and receives the detection information of the air pollution source detected by the air detection main part 12 for calculating, processing and outputting, and the communicator is used for external communication transmission. The detection information of the air detection main part 12 is converted into the detection datum for storage. The communicator 14 receives the air detection datum output by the microprocessor 13, and transmits the air detection datum to a cloud processing device (not shown) or an external device (not shown). Preferably but not exclusively, the external device is a portable mobile device (not shown). The above-mentioned external communication transmission of the communicator 14 can be a wired two-way communication transmission or a wireless two-way communication transmission. Preferably but not exclusively, the wired two-way communication transmission is one selected form the group consisting of a USB communication transmission, a mini-USB communication transmission and a micro-USB communication transmission. Preferably but not exclusively, the wireless two-way communication transmission is one selected from the group consisting of a Wi-Fi communication transmission, a Bluetooth communication transmission, a radio frequency identification communication transmission and a near field communication (NFC) transmission.

In the embodiment, the air pollution source is at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus and a combination thereof.

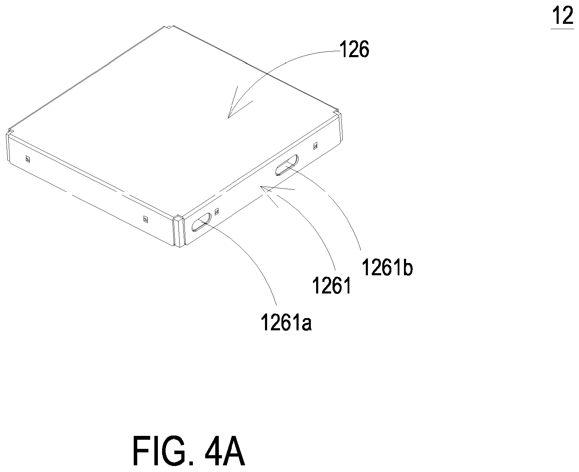

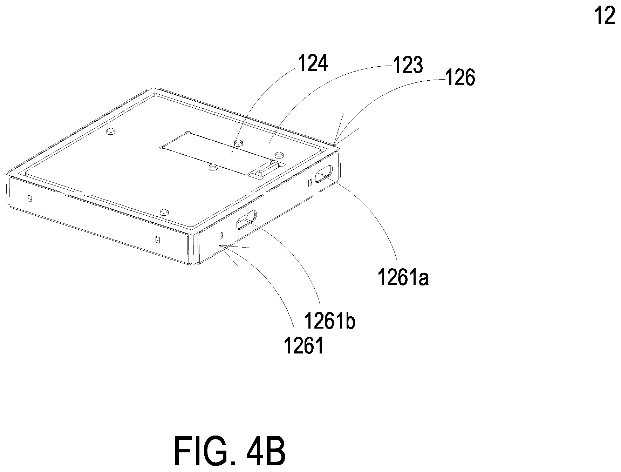

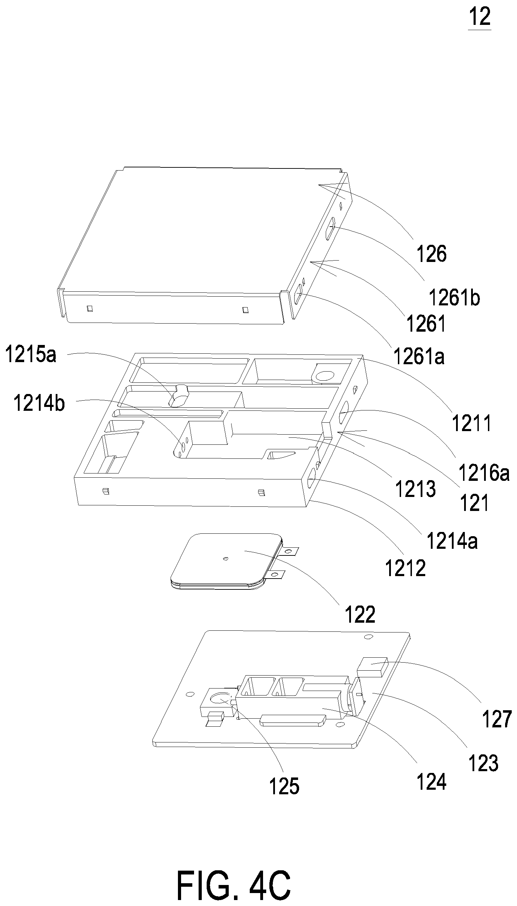

Please refer to FIG. 4A to FIG. 9A. In the embodiment, the air detection main part 12 includes a base 121, a piezoelectric actuator 122, a driving circuit board 123, a laser component 124, a particulate sensor 125, an outer cover 126 and an air sensor 127. In the embodiment, the base 121 includes a first surface 1211, a second surface 1212, a laser loading region 1213, an air-inlet groove 1214, an air-guiding-component loading region 1215 and an air-outlet groove 1216. The first surface 1211 and the second surface 1212 are two surfaces opposite to each other. In the embodiment, the laser loading region 1213 is hollowed out from the first surface 1211 toward the second surface 1212. The outer cover 126 covers the base 121 and includes a side plate 1261. The side plate 1261 has an inlet opening 1261a and an outlet opening 1261b. The air-inlet groove 1214 is concavely formed from the second surface 1212 and disposed adjacent to the laser loading region 1213. The air-inlet groove 1214 includes an air-inlet 1214a and two lateral walls. The air-inlet 1214a is in communication with an environment outside the base 121, and is spatially corresponding in position to an inlet opening 1261a of the outer cover 126. Two transparent windows 1214b are opened on the two lateral walls of the air-inlet groove 1214 and are in communication with the laser loading region 1213. Therefore, the first surface 1211 of the base 121 is covered and attached by the outer cover 126, and the second surface 1212 is covered and attached by the driving circuit board 123, so that an inlet path is defined by the air-inlet groove 1214.

In the embodiment, the air-guiding-component loading region 1215 mentioned above is concavely formed from the second surface 1212 and in communication with the air-inlet groove 1214. A ventilation hole 1215a penetrates a bottom surface of the air-guiding-component loading region 1215. The air-guiding-component loading region 1215 includes four positioning protrusions 1215b disposed at four corners of the air-guiding-component loading region 1215, respectively. In the embodiment, the air-outlet groove 1216 includes an air-outlet 1216a, and the air-outlet 1216a is spatially corresponding to the outlet opening 1261b of the outer cover 126. The air-outlet groove 1216 includes a first section 1216b and a second section 1216c. The first section 1216b is concavely formed out from the first surface 1211 in a region spatially corresponding to a vertical projection area of the air-guiding-component loading region 1215. The second section 1216c is hollowed out from the first surface 1211 to the second surface 1212 in a region where the first surface 1211 is extended from the vertical projection area of the air-guiding-component loading region 1215. The first section 1216b and the second section 1216c are connected to form a stepped structure. Moreover, the first section 1216b of the air-outlet groove 1216 is in communication with the ventilation hole 1215a of the air-guiding-component loading region 1215, and the second section 1216c of the air-outlet groove 1216 is in communication with the air-outlet 1216a. In that, when first surface 1211 of the base 121 is attached and covered by the outer cover 126 and the second surface 1212 of the base 121 is attached and covered by the driving circuit board 123, the air-outlet groove 1216 and the driving circuit board 123 collaboratively define an outlet path.

In the embodiment, the laser component 124 and the particulate sensor 125 are disposed on and electrically connected to the driving circuit board 123 and located within the base 121. In order to clearly describe and illustrate the positions of the laser component 124 and the particulate sensor 125 in the base 121, the driving circuit board 123 is intentionally omitted. The laser component 124 is accommodated in the laser loading region 1213 of the base 121, and the particulate sensor 125 is accommodated in the air-inlet groove 1214 of the base 121 and is aligned to the laser component 124. In addition, the laser component 124 is spatially corresponding to the transparent window 1214b, therefore, a light beam emitted by the laser component 124 passes through the transparent window 1214b and is irradiated into the air-inlet groove 1214. A light beam path emitted from the laser component 124 passes through the transparent window 1214b and extends in an orthogonal direction perpendicular to the air-inlet groove 1214. In the embodiment, a projecting light beam emitted from the laser component 124 passes through the transparent window 1214b and enters the air-inlet groove 1214 to irradiate the suspended particles contained in the air passing through the air-inlet groove 1214. When the suspended particles contained in the air are irradiated and generate scattered light spots, the scattered light spots are received and calculated by the particulate sensor 125 to obtain the air detection information. In the embodiment, the air sensor 127 is positioned and disposed on the driving circuit board 123, electrically connected to the driving circuit board 123, and accommodated in the air-outlet groove 1216, so as to detect the air pollution source introduced into the air-outlet groove 1216. Preferably but not exclusively, in an embodiment, the air sensor 127 includes a volatile-organic-compound sensor for detecting the information of carbon dioxide (CO₂) or volatile organic compounds (TVOC). Preferably but not exclusively, in an embodiment, the air sensor 127 includes a formaldehyde sensor for detecting the information of formaldehyde (HCHO) gas. Preferably but not exclusively, in an embodiment, the air sensor 127 includes a bacteria sensor for detecting the information of bacteria or fungi. Preferably but not exclusively, in an embodiment, the air sensor 127 includes a virus sensor for detecting the information of virus in the air. Preferably but not exclusively, the air sensor 127 is a temperature and humidity sensor for detecting the temperature and humidity information of the air.

In the embodiment, the piezoelectric actuator 122 is accommodated in the square-shaped air-guiding-component loading region 1215 of the base 121. In addition, the air-guiding-component loading region 1215 of the base 121 is in fluid communication with the air-inlet groove 1214. When the piezoelectric actuator 122 is enabled, the air in the air-inlet groove 1214 is inhaled by the piezoelectric actuator 122, so that the air flows into the piezoelectric actuator 122, and is transported into the air-outlet groove 1216 through the ventilation hole 1215a of the air-guiding-component loading region 1215. Moreover, the driving circuit board 123 covers the second surface 1212 of the base 121, and the laser component 124 is positioned and disposed on the driving circuit board 123, and is electrically connected to the driving circuit board 123. The particulate sensor 125 is also positioned and disposed on the driving circuit board 123 and electrically connected to the driving circuit board 123. In that, when the outer cover 126 covers the base 121, the inlet opening 1261a is spatially corresponding to the air-inlet 1214a of the base 121, and the outlet opening 1261b is spatially corresponding to the air-outlet 1216a of the base 121.

In the embodiment, the piezoelectric actuator 122 includes an air-injection plate 1221, a chamber frame 1222, an actuator element 1223, an insulation frame 1224 and a conductive frame 1225. In the embodiment, the air-injection plate 1221 is made by a flexible material and includes a suspension plate 1221a and a hollow aperture 1221b. The suspension plate 1221a is a sheet structure and is permitted to undergo a bending deformation. Preferably but not exclusively, the shape and the size of the suspension plate 1221a are accommodated in the inner edge of the air-guiding-component loading region 1215, but not limited thereto. The hollow aperture 1221b passes through a center of the suspension plate 1221a, so as to allow the air to flow therethrough. Preferably but not exclusively, in the embodiment, the shape of the suspension plate 1221a is selected from the group consisting of a square, a circle, an ellipse, a triangle and a polygon, but not limited thereto.

In the embodiment, the chamber frame 1222 is carried and stacked on the air-injection plate 1221. In addition, the shape of the chamber frame 1222 is corresponding to the air-injection plate 1221. The actuator element 1223 is carried and stacked on the chamber frame 1222. A resonance chamber 1226 is collaboratively defined by the actuator element 1223, the chamber frame 1222 and the suspension plate 1221a and is formed between the actuator element 1223, the chamber frame 1222 and the suspension plate 1221a. The insulation frame 1224 is carried and stacked on the actuator element 1223 and the appearance of the insulation frame 1224 is similar to that of the chamber frame 1222. The conductive frame 1225 is carried and stacked on the insulation frame 1224, and the appearance of the conductive frame 1225 is similar to that of the insulation frame 1224. In addition, the conductive frame 1225 includes a conducting pin 1225a and a conducting electrode 1225b. The conducting pin 1225a is extended outwardly from an outer edge of the conductive frame 1225, and the conducting electrode 1225b is extended inwardly from an inner edge of the conductive frame 1225.

Moreover, the actuator element 1223 further includes a piezoelectric carrying plate 1223a, an adjusting resonance plate 1223b and a piezoelectric plate 1223c. The piezoelectric carrying plate 1223a is carried and stacked on the chamber frame 1222. The adjusting resonance plate 1223b is carried and stacked on the piezoelectric carrying plate 1223a. The piezoelectric plate 1223c is carried and stacked on the adjusting resonance plate 1223b. The adjusting resonance plate 1223b and the piezoelectric plate 1223c are accommodated in the insulation frame 1224. The conducting electrode 1225b of the conductive frame 1225 is electrically connected to the piezoelectric plate 1223c. In the embodiment, the piezoelectric carrying plate 1223a and the adjusting resonance plate 1223b are made by a conductive material. The piezoelectric carrying plate 1223a includes a piezoelectric pin 1223d. The piezoelectric pin 1223d and the conducting pin 1225a are electrically connected to a driving circuit (not shown) of the driving circuit board 123, so as to receive a driving signal, such as a driving frequency and a driving voltage. Through this structure, a circuit is formed by the piezoelectric pin 1223d, the piezoelectric carrying plate 1223a, the adjusting resonance plate 1223b, the piezoelectric plate 1223c, the conducting electrode 1225b, the conductive frame 1225 and the conducting pin 1225a for transmitting the driving signal. Moreover, the insulation frame 1224 is insulated between the conductive frame 1225 and the actuator element 1223, so as to avoid the occurrence of a short circuit. Thereby, the driving signal is transmitted to the piezoelectric plate 1223c. After receiving the driving signal such as the driving frequency and the driving voltage, the piezoelectric plate 1223c deforms due to the piezoelectric effect, and the piezoelectric carrying plate 1223a and the adjusting resonance plate 1223b are further driven to generate the bending deformation in the reciprocating manner.

Furthermore, in the embodiment, the adjusting resonance plate 1223b is located between the piezoelectric plate 1223c and the piezoelectric carrying plate 1223a and served as a cushion between the piezoelectric plate 1223c and the piezoelectric carrying plate 1223a. Thereby, the vibration frequency of the piezoelectric carrying plate 1223a is adjustable. Basically, the thickness of the adjusting resonance plate 1223b is greater than the thickness of the piezoelectric carrying plate 1223a, and the vibration frequency of the actuator element 1223 can be adjusted by adjusting the thickness of the adjusting resonance plate 1223b. In the embodiment, the air-injection plate 1221, the chamber frame 1222, the actuator element 1223, the insulation frame 1224 and the conductive frame 1225 are stacked and positioned in the air-guiding-component loading region 1215 sequentially, so that the piezoelectric actuator 122 is supported and positioned in the air-guiding-component loading region 1215. A plurality of clearances 1221c are defined between the suspension plate 1221a of the air-injection plate 1221 and an inner edge of the air-guiding-component loading region 1215 for air flowing therethrough.

A flowing chamber 1227 is formed between the air-injection plate 1221 and the bottom surface of the air-guiding-component loading region 1215. The flowing chamber 1227 is in communication with the resonance chamber 1226 between the actuator element 1223, the chamber frame 1222 and the suspension plate 1221a through the hollow aperture 1221b of the air-injection plate 1221. By controlling the vibration frequency of the air in the resonance chamber 1226 to be close to the vibration frequency of the suspension plate 1221a, the Helmholtz resonance effect is generated between the resonance chamber 1226 and the suspension plate 1221a, so as to improve the efficiency of air transportation. When the piezoelectric plate 1223c is moved away from the bottom surface of the air-guiding-component loading region 1215, the suspension plate 1221a of the air-injection plate 1221 is driven to move away from the bottom surface of the air-guiding-component loading region 1215 by the piezoelectric plate 1223c. In that, the volume of the flowing chamber 1227 is expanded rapidly, the internal pressure of the flowing chamber 1227 is decreased to form a negative pressure, and the air outside the piezoelectric actuator 122 is inhaled through the clearances 1221c and enters the resonance chamber 1226 through the hollow aperture 1221b. Consequently, the pressure in the resonance chamber 1226 is increased to generate a pressure gradient. When the suspension plate 1221a of the air-injection plate 1221 is driven by the piezoelectric plate 1223c to move toward the bottom surface of the air-guiding-component loading region 1215, the air in the resonance chamber 1226 is discharged out rapidly through the hollow aperture 1221b, and the air in the flowing chamber 1227 is compressed, thereby the converged air is quickly and massively ejected out of the flowing chamber 1227 under the condition close to an ideal air state of the Benulli's law, and transported to the ventilation hole 1215a of the air-guiding-component loading region 1215.

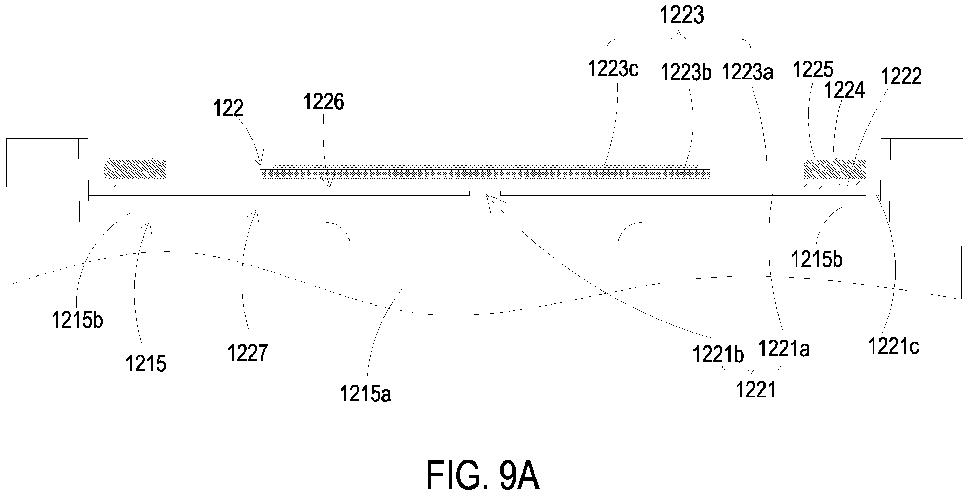

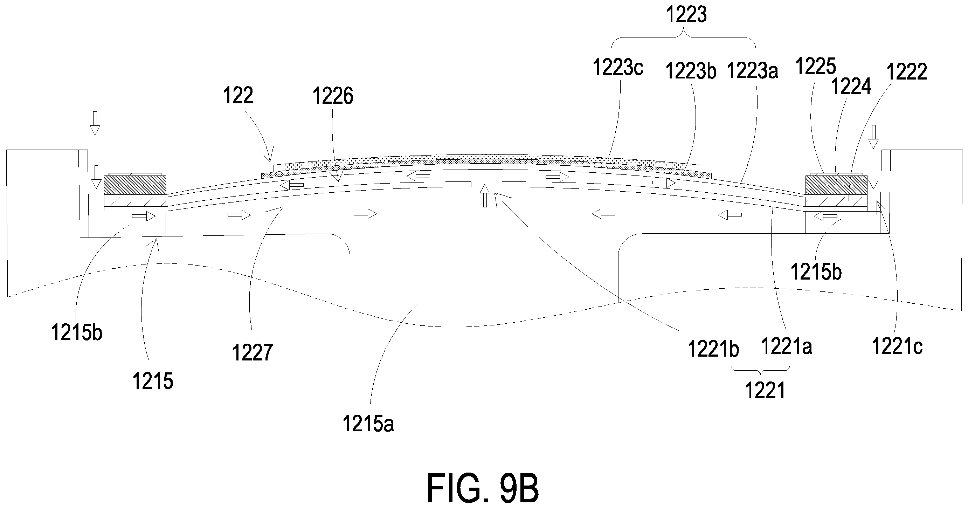

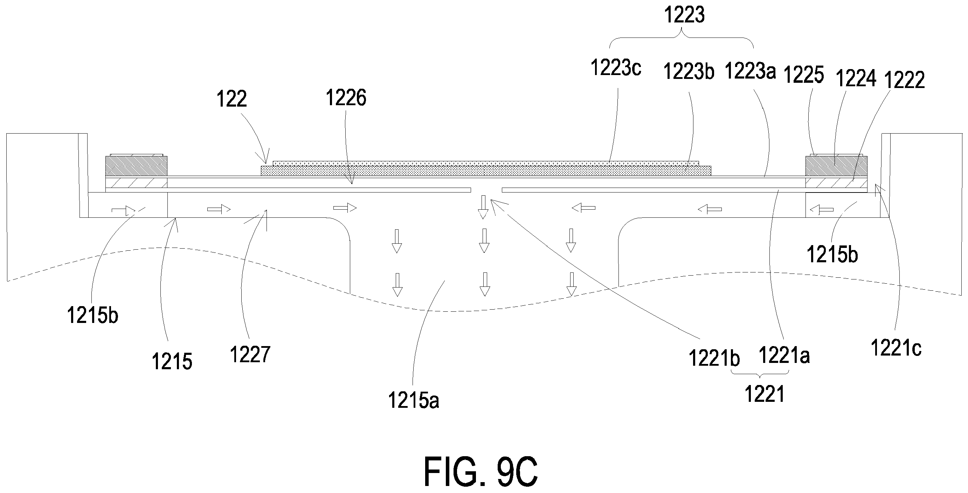

By repeating the above operation steps shown in FIG. 9B and FIG. 9C, the piezoelectric plate 1223c is driven to generate the bending deformation in a reciprocating manner. According to the principle of inertia, since the air pressure inside the resonance chamber 1226 is lower than the equilibrium air pressure after the converged air is ejected out, the air is introduced into the resonance chamber 1226 again. Moreover, the vibration frequency of the air in the resonance chamber 1226 is controlled to be close to the vibration frequency of the piezoelectric plate 1223c, so as to generate the Helmholtz resonance effect to achieve the air transportation at high speed and in large quantities.

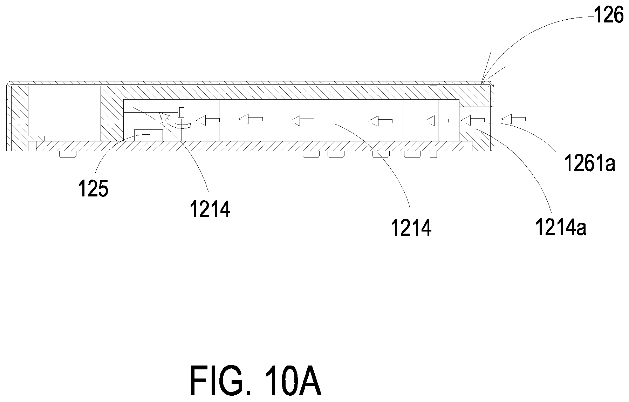

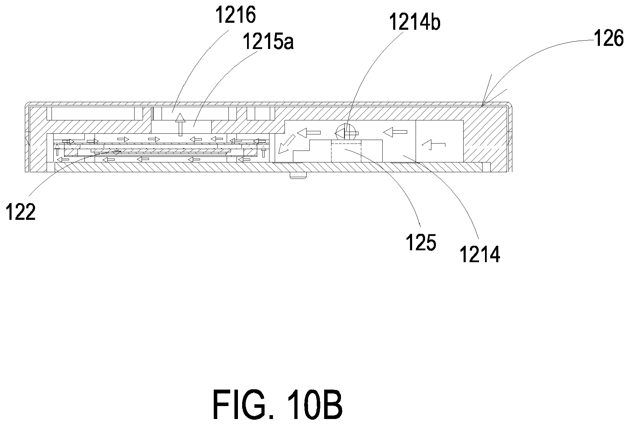

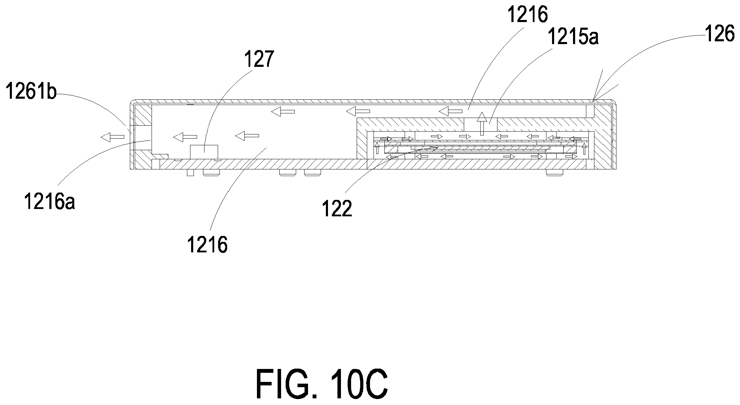

Please refer to FIG. 10A to FIG. 10C. The air is inhaled through the air-inlet 1214a on the outer cover 126, flows into the air-inlet groove 1214 of the base 121 through the air-inlet 1214a, and is transported to the position of the particulate sensor 125. The piezoelectric actuator 122 is enabled continuously to inhale the air into the inlet path, and facilitate the air outside the air detection module to be introduced rapidly, flow stably, and transported above the particulate sensor 125. At this time, a projecting light beam emitted from the laser component 124 passes through the transparent window 1214b to irritate the suspended particles contained in the air flowing above the particulate sensor 125 in the air-inlet groove 1214. When the suspended particles contained in the air are irradiated and generate scattered light spots, the scattered light spots are received and calculated by the particulate sensor 125 for obtaining related information about the sizes and the concentration of the suspended particles contained in the air. Moreover, the air above the particulate sensor 125 is continuously driven and transported by the piezoelectric actuator 122, flows into the ventilation hole 1215a of the air-guiding-component loading region 1215, and is transported to the air-outlet groove 1216. At last, after the air flows into the air outlet groove 1216, the air is continuously transported into the air-outlet groove 1216 by the piezoelectric actuator 122, and thus the air in the air-outlet groove 1216 is pushed to discharge through the air-outlet 1216a and the outlet opening 1261b.

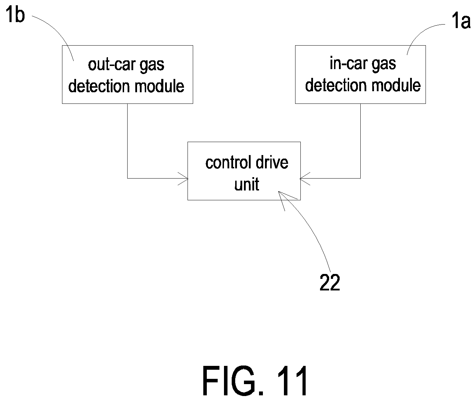

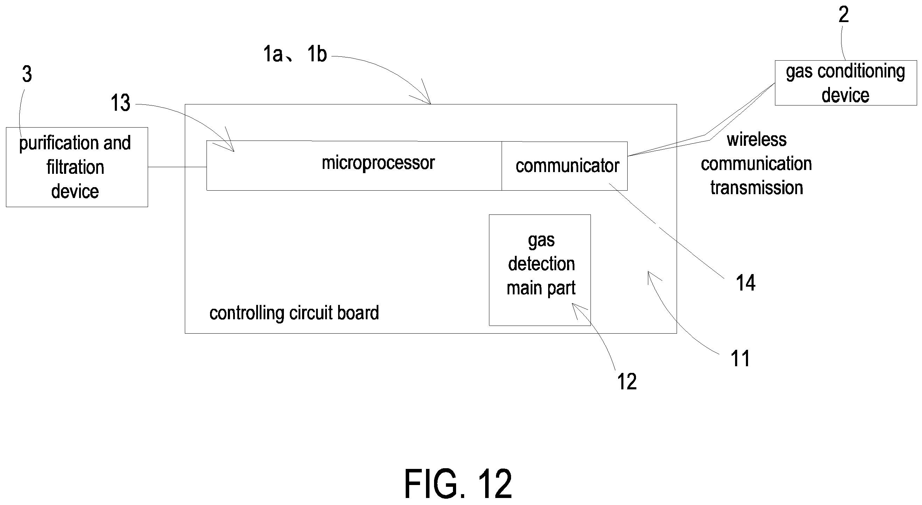

Accordingly, in view of the above description of the present disclosure, as shown in FIG. 11 and FIG. 12, the plurality of in-car air detection modules 1a, the plurality of out-car air detection modules 1b, the air conditioning device 2 and the plurality of filtering and purification components D are installed in a car. The control drive unit 22 of the air conditioning device 2 receives and compares the air detection data of the plurality of in-car air detection modules 1a and a plurality of out-car air detection modules 1b under the calculation of artificial intelligence, so as to locate the position of the air pollution source in the interior space A of the car. Moreover, the filtering and purification component D and the purification and filtration device 3 disposed adjacent to the location of the air pollution source are intelligently selected and controlled to be enabled to execute the filtering process, so as to provide clean, safe and breathable air in the interior space A of the car.

## Claims

1. An in-car air pollution prevention system for exchanging and filtering air pollution source in an interior space (A) of a car, comprising:
a plurality of air detection modules (1a, 1b) for detecting a status of air and the air pollution source, and transmitting at least one air detection datum;
an air conditioning device (2) for controlling the introduction or not introduction of external air in an exterior space (B) outside the car into the interior space (A) of the car and the exhalation of the air pollution in the interior space (A) of the car into the exterior space (B) outside the car, wherein the air conditioning device (2) comprises a ventilation channel (21), a control drive unit (22) and an air guider (C), wherein the ventilation channel (21) includes at least one air outlet (211), at least one air inlet (212) and an external-air inlet (213) and the air guider (C) is disposed within the ventilation channel (21), wherein the control drive unit (22) receives and compares the air detection data outputted by the air detection modules (1a, 1b), and the air guider (C) performs the discharging of the air from the at least one air outlet (211); and
a plurality of filtering and purification components (D), wherein at least one of the plurality of filtering and purification components (D) is disposed at the at least one air outlet (211) for filtering and purifying the external air and the air pollution source, and at least one of the plurality of filtering and purification components (D) is disposed at the at least one air inlet (212) for filtering and purifying the air pollution source;
wherein after the control drive unit (22) compares the air detection data, the air conditioning device (2) is allowed to intelligently select and control the introduction or not introduction of the external air in the exterior space (B) outside the car into the interior space (A) of the car, and the air guider (C) of the air conditioning device (2) is instantly controlled to be enable by the plurality of air detection modules (1a, 1b) in a state of a monitoring mechanism for filtering and purifying the air pollution source in the interior space (A) of the car through the plurality of filtering and purification components (D), so that the air pollution source in the interior space (A) of the car is filtered and exchanged to provide clean air,
**characterized in that**
the air conditioning device (2) further comprises an intake-control element (24), the external-air inlet (213) is used to introduce the external air in the exterior space (B) outside the car, and the air inlet (212) is used to introduce the air pollution source in the interior space (A) of the car, wherein the intake-control element (24) is controlled by the control drive unit (22) to select the opening of the external-air inlet (213), and the control drive unit (22) receives and compares the out-car air detection datum outputted by the out-car air detection module (1b) and the in-car air detection datum output by the in-car air detection module (1a) to determine if the external-air inlet (213) need to be opened, so as to control the introduction or non-introduction of the external air in the exterior space (B) outside the car or the inhalation of the air pollution source in the interior space (A) of the car through the air inlet (212), and
the plurality of air detection modules (1a, 1b) comprises at least one out-car air detection module (1b) and at least one in-car air detection module (1a), the at least one out-car air detection module (1b) is disposed in the external-air inlet (213) for detecting the external air in the exterior space (B) outside the car and transmitting an out-car air detection datum, and the at least one in-car air detection module (1a) is disposed in the interior space (A) of the car for detecting the air pollution source in the interior space (A) of the car and transmitting at least one in-car air detection datum, and **in that**
the control drive unit (22) receives and compares the in-car air detection data outputted by at least three in-car air detection modules (1a) under the calculation of artificial intelligence, so as to locate the position of the air pollution source in the interior space (A) of the car, and intelligently select and control the air outlet adjacent to the air pollution source to expel air in first priority, and direct the air pollution source toward the air inlet (212) adjacent thereto, wherein the control drive unit (22) of the air conditioning device (2) selects and controls the rest of the air outlets (212) to expel air under the calculation of artificial intelligence, so that an airflow is generated to direct the air pollution source toward the air inlet (212) adjacent to the air pollution source to be inhaled for filtering rapidly.

2. The in-car air pollution prevention system according to claim 1, wherein the air pollution source is at least one selected from the group consisting of suspended particles, carbon monoxide, carbon dioxide, ozone, sulfur dioxide, nitrogen dioxide, lead, total volatile organic compounds (TVOC), formaldehyde, bacteria, fungi, virus and a combination thereof.

3. The in-car air pollution prevention system according to claim 1, wherein the filtering and purification component (D) comprises an activated carbon (D1) and a high efficiency particulate air (HEPA) filter screen (D2), wherein the state of the monitoring mechanism is enabled when at least one of the air detection data of the air pollution source detected by the plurality of air detection modules in the interior space (A) of the car is greater than a safe detection value.

4. The in-car air pollution prevention system according to claim 3, wherein the safe detection value is at least one selected from the group consisting of PM2.5 less than 35 µg/m³, carbon dioxide content less than 1000 ppm, total volatile organic compounds (TVOC) less than 0.56 ppm, formaldehyde content less than 0.08 ppm, the amount of bacteria less than 1500 CFU/m³, the amount of fungi less than 1000 CFU/m³, sulfur dioxide content less than 0.075 ppm, nitrogen dioxide content less than 0.1 ppm, carbon monoxide content less than 9 ppm, ozone content less than 0.06 ppm, lead content less than 0.15 µg/m³ and a combination thereof.

5. The in-car air pollution prevention system according to claim 1, wherein the control drive unit (22) receives and compares the in-car air detection data outputted by at least three in-car air detection modules (1a) under the calculation of artificial intelligence, so as to locate the position of the air pollution source in the interior space (A) of the car, and intelligently select and control the filtering and purification component (D) disposed in the position of the air inlet (212) adjacent to the air pollution source to accelerate the transportation for filtering.

6. The in-car air pollution prevention system according to claim 1, further comprising at least one purification and filtration device (3), and the purification and filtration device (3) includes the air guider (C) and the filtering and purification component (D), and the in-car air detection module (1a) is in combination with the purification and filtration device (3) to control the enablement of the air guider (C), so that the air pollution source in the interior space (A) of the car is guided to enter the filtering and purification component (D) of the purification and filtration device (3) for filtering and purifying.

7. The in-car air pollution prevention system according to claim 6, wherein the control drive unit (22) of the air conditioning device (2) receives and compares the in-car air detection data outputted by at least three in-car air detection modules (1a) under the calculation of artificial intelligence, so as to locate the position of the air pollution source in the interior space (A) of the car, and intelligently select and control the enablement of the purification and filtration device (3) adjacent to the air pollution source, so that the air pollution source can be inhaled into the purification and filtration device (3) without diffusion and accelerate the filtration.

8. The in-car air pollution prevention system according to claim 6, wherein the control drive unit (22) of the air conditioning device (2) receives and compares the in-car air detection data outputted by at least three in-car air detection modules (1a) under the calculation of artificial intelligence, so as to locate the position of the air pollution source in the interior space (A) of the car, and intelligently select and control the purification and filtration device (3) adjacent to the air pollution source to be enabled in first priority, wherein the control drive unit (22) of the air conditioning device (2) selects and controls the rest of the purification and filtration devices (3) to be enabled under the calculation of artificial intelligence, so that an airflow is generated to direct the air pollution source toward the purification and filtration device (3) adjacent to the air pollution source to be inhaled for filtering rapidly.

9. The in-car air pollution prevention system according to claim 6, wherein the at least one purification and filtration device (3) is in combination with and embedded on a trim panel, a seat or a door pillar in the interior space (A) of the car, and the in-car air detection module (1a) transmits the in-car air detection datum for being received and compared by the control drive unit (22) of the air conditioning device (2) under the calculation of artificial intelligence, and intelligently selecting and controlling the enablement of the purification and filtration device (3) adjacent to the air pollution source.

10. The in-car air pollution prevention system according to claim 6, wherein the in-car air detection module (2) is in combination with a wearable device worn on a human body to detect the air pollution source in the interior space (A) of the car in real-time, and transmit the in-car air detection datum in the interior space (A) of the car for receiving and comparing by the control drive unit (22) of the air conditioning device (2) under the calculation of artificial intelligence, and intelligently selecting and controlling the enablement of the purification and filtration device (3) adjacent to the air pollution source.

11. The in-car air pollution prevention system according to claim 6, wherein at least one of the plurality of in-car air detection modules (1a) is disposed at two sides of the plurality of purification and filtration devices (3), so that the control drive unit (22) receives and compares the in-car air detection data outputted by the in-car air detection modules (1a) at the positions of the plurality of the purification and filtration devices (3), so as to make sure that the air pollution source is filtered by the plurality of the purification and filtration devices (3) to generate clean air introduced into the interior space (A) of the car.

12. The in-car air pollution prevention system according to claim 3, wherein the service life of the HEPA filter screen (D2) is calculated based on the monitoring mechanism of the air detection data detected by the in-car air detection module (1a) and the out-car air detection module (1b) with reference to an accumulated operation time of the air guider (C) in the air conditioning device (2).

13. The in-car air pollution prevention system according to claim 1, wherein the control drive unit (22) comprises a touch screen (221) for setting up the control instructions of the air conditioning device (2) by touching the touch screen (221) and displaying the air detection datum.

## Patentansprüche

1. In-Car-Luftverschmutzungsverhinderungssystem zum Austauschen und Filtern einer Luftverschmutzungsquelle im Innenraum (A) eines Fahrzeugs, umfassend:
eine Mehrzahl von Luftdetektionsmodulen (1a, 1b) zum Detektieren eines Zustands der Luft und der Luftverschmutzungsquelle, und zum Übertragen von mindestens einem Luftdetektionswert;
eine Luftkonditionierungsvorrichtung (2) zum Steuern des Einleitens oder Nicht-Einleitens von Außenluft in einem Außenraum (B) außerhalb des Fahrzeugs in den Innenraum (A) des Fahrzeugs und des Ausblasens der Luftverschmutzung im Innenraum (A) des Fahrzeugs in den Außenraum (B) außerhalb des Fahrzeugs, wobei die Luftkonditionierungsvorrichtung (2) einen Ventilationskanal (21), eine Steuerantriebseinheit (22) und eine Luftleiteinrichtung (C) umfasst, wobei der Ventilationskanal (21) mindestens einen Luftauslass (211), mindestens einen Lufteinlass (212) und einen Außenlufteinlass (213) aufweist und die Luftleiteinrichtung (C) in dem Ventilationskanal (21) angeordnet ist, wobei die Steuerantriebseinheit (22) die von den Luftdetektionsmodulen (1a, 1b) ausgegebenen Luftdetektionswerte empfängt und vergleicht, und die Luftleiteinrichtung (C) das Ausstoßen der Luft aus dem mindestens einen Luftauslass (211) durchführt; und
eine Mehrzahl von Filter- und Reinigungskomponenten (D), wobei mindestens eine der Mehrzahl von Filter- und Reinigungskomponenten (D) an dem mindestens einen Luftauslass (211) angeordnet ist, um die Außenluft und die Luftverschmutzungsquelle zu filtern und zu reinigen, und mindestens eine der Mehrzahl von Filter- und Reinigungskomponenten (D) an dem mindestens einen Lufteinlass (212) angeordnet ist, um die Luftverschmutzungsquelle zu filtern und zu reinigen;
wobei, nachdem die Steuerantriebseinheit (22) die Luftdetektionswerte verglichen hat, es der Luftkonditionierungsvorrichtung (2) ermöglicht wird, das Einleiten oder Nicht-Einleiten der Außenluft im Außenraum (B) außerhalb des Fahrzeugs in den Innenraum (A) des Fahrzeugs intelligent auszuwählen und zu steuern, und die Luftleiteinrichtung (C) der Luftkonditionierungsvorrichtung (2) augenblicklich gesteuert wird, um durch die Mehrzahl von Luftdetektionsmodulen (1a, 1b) in einen Zustand eines Überwachungsmechanismus zum Filtern und Reinigen der Luftverschmutzungsquelle im Innenraum (A) des Fahrzeugs durch die Mehrzahl von Filter- und Reinigungskomponenten (D) aktiviert zu werden, so dass die Luftverschmutzungsquelle im Innenraum (A) des Fahrzeugs gefiltert und ausgetauscht wird, um saubere Luft zur Verfügung zu stellen,
**dadurch gekennzeichnet, dass**
die Luftkonditionierungsvorrichtung (2) außerdem ein Einlasssteuerelement (24) aufweist, der Außenlufteinlass (213) verwendet wird, um die Außenluft im Außenraum (B) außerhalb des Fahrzeugs einzuleiten, und der Lufteinlass (212) verwendet wird, um die Luftverschmutzungsquelle im Innenraum (A) des Fahrzeugs einzuleiten, wobei das Einlasssteuerelement (24) durch die Steuerantriebseinheit (22) gesteuert wird, um das Öffnen des Außenlufteinlass (213) auszuwählen, und die Steuerantriebseinheit (22) den vom Out-Car-Luftdetektionsmodul (1b) ausgegebenen Out-Car-Luftdetektionswert und den vom In-Car-Luftdetektionsmodul (1a) ausgegebenen In-Car-Luftdetektionswert empfängt und vergleicht, um zu bestimmen, ob der Außenlufteinlass (213) geöffnet werden muss, um so das Einleiten oder Nicht-Einleiten der Außenluft im Außenraum (B) außerhalb des Fahrzeugs oder das Einsaugen der Luftverschmutzungsquelle im Innenraum (A) des Fahrzeugs durch den Lufteinlass (212) zu steuern, und
die Mehrzahl von Luftdetektionsmodulen (1a, 1b) mindestens ein Out-Car-Luftdetektionsmodul (1b) und mindestens ein In-Car-Luftdetektionsmodul (1a) umfasst, das mindestens eine Out-Car-Luftdetektionsmodul (1b) in dem Außenlufteinlass (213) angeordnet ist, um die Außenluft im Außenraum (B) außerhalb des Fahrzeugs zu detektieren und einen Out-Car-Luftdetektionswert zu übertragen, und das mindestens eine In-Car-Luftdetektionsmodul (1a) im Innenraum (A) des Fahrzeugs angeordnet ist, um die Luftverschmutzungsquelle im Innenraum (A) des Fahrzeugs zu detektieren und mindestens einen In-Car-Luftdetektionswert zu übertragen, und dass
die Steuerantriebseinheit (22) die von mindestens drei In-Car-Luftdetektionsmodulen (1a) ausgegebenen In-Car-Luftdetektionswerte unter Berechnung künstlicher Intelligenz empfängt und vergleicht, um so die Position der Luftverschmutzungsquelle im Innenraum (A) des Fahrzeugs zu lokalisieren, und den Luftauslass benachbart zur Luftverschmutzungsquelle intelligent auszuwählen und zu steuern, um in erster Priorität Luft auszustoßen, und die Luftverschmutzungsquelle in Richtung des dazu benachbarten Lufteinlass (212) zu leiten, wobei die Steuerantriebseinheit (22) der Luftkonditionierungsvorrichtung (2) die übrigen Luftauslässe (212) auswählt und steuert, um Luft unter Berechnung künstlicher Intelligenz auszustoßen, so dass ein Luftstrom erzeugt wird, um die Luftverschmutzungsquelle in Richtung des Lufteinlass (212) benachbart zur Luftverschmutzungsquelle zu leiten, um zwecks Filterung schnell eingesaugt zu werden.

2. In-Car-Luftverschmutzungsverhinderungssystem nach Anspruch 1, wobei die Luftverschmutzungsquelle mindestens eine ist, die aus der Gruppe ausgewählt ist, die schwebende Partikel, Kohlenmonoxid, Kohlendioxid, Ozon, Schwefeldioxid, Stickstoffdioxid, Blei, gesamte flüchtige organische Verbindungen (TVOC), Formaldehyd, Bakterien, Pilze, Viren und eine Kombination davon enthält.

3. In-Car-Luftverschmutzungsverhinderungssystem nach Anspruch 1, wobei die Filter- und Reinigungskomponente (D) einen Aktivkohlefilter (D1) und ein HEPA-Filtersieb (High Efficiency Particulate Air Filtersieb) (D2) umfasst, wobei der Zustand des Überwachungsmechanismus aktiviert wird, wenn mindestens einer der Luftdetektionswerte der Luftverschmutzungsquelle, die durch die Mehrzahl von Luftdetektionsmodulen im Innenraum (A) des Fahrzeugs detektiert werden, größer als ein sicherer Detektionswert ist.

4. In-Car-Luftverschmutzungsverhinderungssystem nach Anspruch 3, wobei der sichere Detektionswert mindestens einer ist, der aus der Gruppe ausgewählt ist, die PM2,5 von weniger als 35 µg/m³, einen Kohlendioxidgehalt von weniger als 1000 ppm, gesamte flüchtige organische Verbindungen (TVOC) von weniger als 0,56 ppm, einen Formaldehydgehalt von weniger als 0,08 ppm, eine Menge an Bakterien von weniger als 1500 KBE/m³, eine Menge an Pilzen von weniger als 1000 KBE/m³, einen Schwefeldioxidgehalt von weniger als 0,075 ppm, einen Stickstoffdioxidgehalt von weniger als 0,1 ppm, einen Kohlenmonoxidgehalt von weniger als 9 ppm, einen Ozongehalt von weniger als 0,06 ppm, einen Bleigehalt von weniger als 0,15 µg/m³ und eine Kombination davon enthält.

5. In-Car-Luftverschmutzungsverhinderungssystem nach Anspruch 1, wobei die Steuerantriebseinheit (22) die von mindestens drei In-Car-Luftdetektionsmodulen (1a) ausgegebenen In-Car-Luftdetektionswerte unter Berechnung künstlicher Intelligenz empfängt und vergleicht, um so die Position der Luftverschmutzungsquelle im Innenraum (A) des Fahrzeugs zu lokalisieren, und die Filter- und Reinigungskomponente (D), die an der Position des Lufteinlass (212) benachbart zur Luftverschmutzungsquelle angeordnet ist, intelligent auszuwählen und zu steuern, um den Transport zwecks Filterung zu beschleunigen.

6. In-Car-Luftverschmutzungsverhinderungssystem nach Anspruch 1, ferner umfassend mindestens eine Reinigungs- und Filtervorrichtung (3), wobei die Reinigungs- und Filtervorrichtung (3) die Luftleiteinrichtung (C) sowie die Filter- und Reinigungskomponente (D) enthält, und das In-Car-Luftdetektionsmodul (1a) mit der Reinigungs- und Filtervorrichtung (3) in Kombination steht, um die Aktivierung der Luftleiteinrichtung (C) zu steuern, so dass die Luftverschmutzungsquelle im Innenraum (A) des Fahrzeugs geleitet wird, um in die Filter- und Reinigungskomponente (D) der Reinigungs- und Filtervorrichtung (3) einzutreten, um gefiltert und gereinigt zu werden.

7. In-Car-Luftverschmutzungsverhinderungssystem nach Anspruch 6, wobei die Steuerantriebseinheit (22) der Luftkonditionierungsvorrichtung (2) die von mindestens drei In-Car-Luftdetektionsmodulen (1a) ausgegebenen In-Car-Luftdetektionswerte unter Berechnung künstlicher Intelligenz empfängt und vergleicht, um so die Position der Luftverschmutzungsquelle im Innenraum (A) des Fahrzeugs zu lokalisieren, und die Aktivierung der Reinigungs- und Filtervorrichtung (3) benachbart zur Luftverschmutzungsquelle intelligent auszuwählen und zu steuern, so dass die Luftverschmutzungsquelle ohne Diffusion in die Reinigungs- und Filtervorrichtung (3) eingesaugt werden kann und die Filterung beschleunigt wird.

8. In-Car-Luftverschmutzungsverhinderungssystem nach Anspruch 6, wobei die Steuerantriebseinheit (22) der Luftkonditionierungsvorrichtung (2) die von mindestens drei In-Car-Luftdetektionsmodulen (1a) ausgegebenen In-Car-Luftdetektionswerte unter Berechnung künstlicher Intelligenz empfängt und vergleicht, um so die Position der Luftverschmutzungsquelle im Innenraum (A) des Fahrzeugs zu lokalisieren, und die Reinigungs- und Filtervorrichtung (3) benachbart zur Luftverschmutzungsquelle intelligent auszuwählen und zu steuert, um mit erster Priorität aktiviert zu werden, wobei die Steuerantriebseinheit (22) der Luftkonditionierungsvorrichtung (2) die übrigen Reinigungs- und Filtervorrichtungen (3) auswählt und steuert, um unter Verwendung künstlicher Intelligenz aktiviert zu werden, so dass ein Luftstrom erzeugt wird, um die Luftverschmutzungsquelle in Richtung der Reinigungs- und Filtervorrichtung (3) benachbart zur Luftverschmutzungsquelle zu leiten, um zwecks Filterung schnell eingesaugt zu werden.

9. In-Car-Luftverschmutzungsverhinderungssystem nach Anspruch 6, wobei die mindestens eine Reinigungs- und Filtervorrichtung (3) mit einer Verkleidung, einem Sitz oder einer Türsäule im Innenraum (A) des Fahrzeugs in Kombination steht und darin eingebettet ist, und das In-Car-Luftdetektionsmodul (1a) die In-Car-Luftdetektionswerte überträgt, um von der Steuerantriebseinheit (22) der Luftkonditionierungsvorrichtung (2) unter Verwendung künstlicher Intelligenz empfangen und verglichen werden, und die Aktivierung der Reinigungs- und Filtervorrichtung (3) benachbart zur Luftverschmutzungsquelle intelligent auswählt und steuert.

10. In-Car-Luftverschmutzungsverhinderungssystem nach Anspruch 6, wobei das In-Car-Luftdetektionsmodul (2) mit einer tragbaren Vorrichtung, die an einem menschlichen Körper getragen wird, in Kombination steht, um die Luftverschmutzungsquelle im Innenraum (A) des Fahrzeugs in Echtzeit zu detektieren, und den In-Car-Luftdetektionswert im Innenraum (A) des Fahrzeugs zu übertragen, um von der Steuerantriebseinheit (22) der Luftkonditionierungsvorrichtung (2) unter Berechnung künstlicher Intelligenz übertragen und verglichen zu werden, die Aktivierung der Reinigungs- und Filtervorrichtung (3) benachbart zur Luftverschmutzungsquelle intelligent auswählt und steuert.

11. In-Car-Luftverschmutzungsverhinderungssystem nach Anspruch 6, wobei mindestens eines der Mehrzahl von In-Car-Luftdetektionsmodule (1a) an zwei Seiten der Mehrzahl von Reinigungs- und Filtervorrichtungen (3) angeordnet ist, so dass die Steuerantriebseinheit (22) die von den In-Car-Luftdetektionsmodulen (1a) an den Positionen der Mehrzahl von Reinigungs- und Filtervorrichtungen (3) ausgegebenen In-Car-Luftdetektionswerte empfängt und vergleicht, um so zu gewährleisten, dass die Luftverschmutzungsquelle durch die Mehrzahl von Reinigungs- und Filtervorrichtungen (3) gefiltert wird, um saubere Luft zu erzeugen, die in den Innenraum (A) des Fahrzeugs eingeleitet wird.

12. In-Car-Luftverschmutzungsverhinderungssystem nach Anspruch 3, wobei die Betriebsdauer des HEPA-Filtersiebs (D2) basierend auf dem Überwachungsmechanismus der von dem In-Car-Luftdetektionsmodul (1a) und dem Out-Car-Luftdetektionsmodul (1b) detektierten Luftdetektionswerte unter Bezugnahme auf eine akkumulierte Betriebszeit der Luftleiteinrichtung (C) in der Luftkonditionierungsvorrichtung (2) berechnet wird.

13. In-Car-Luftverschmutzungsverhinderungssystem nach Anspruch 1, wobei die Steuerantriebseinheit (22) einen Touchscreen (221) zum Einstellen der Steuerbefehle der Luftkonditionierungsvorrichtung (2) durch Berühren des Touchscreen (221) und Anzeigen der Luftdetektionswerte aufweist.

## Revendications

1. Système embarqué de prévention contre la pollution de l'air pour échanger et filtrer une source de pollution de l'air dans un espace intérieur (A) d'une automobile, comprenant:
une pluralité de modules de détection d'air (1a, 1b) pour détecter un état de l'air et de la source de pollution de l'air, et pour transmettre au moins une donnée de détection d'air;
un dispositif de climatisation (2) pour commander l'introduction ou la non introduction d'air externe situé dans un espace extérieur (B) à l'extérieur de l'automobile dans l'espace intérieur (A) de l'automobile et l'expulsion de la pollution de l'air présente dans l'espace intérieur (A) de l'automobile vers l'espace extérieur (B) à l'extérieur de l'automobile, dans lequel le dispositif de climatisation (2) comprend un canal de ventilation (21), une unité de pilotage de commande (22) et un diffuseur d'air (C), dans lequel le canal de ventilation (21) comporte au moins une sortie d'air (211), au moins une entrée d'air (212) et une entrée d'air externe (213) et le diffuseur d'air (C) est placé dans le canal de ventilation (21), dans lequel l'unité de pilotage de commande (22) reçoit et compare les données de détection d'air délivrées par les modules de détection d'air (1a, 1b), et le diffuseur d'air (C) réalise le refoulement de l'air depuis ladite au moins une sortie d'air (211); et
une pluralité de composants de filtration et de purification (D), dans lequel au moins l'un des composants de filtration et de purification (D) est placé au niveau de ladite au moins une sortie d'air (211) pour filtrer et purifier l'air externe et la source de pollution de l'air, et au moins l'un des composants de filtration et de purification (D) est placé au niveau de ladite au moins une entrée d'air (212) pour filtrer et purifier la source de pollution de l'air;
dans lequel, après que l'unité de pilotage de commande (22) a comparé les données de détection d'air, le dispositif de climatisation (2) est autorisé à sélectionner et commander de façon intelligente l'introduction ou la non introduction de l'air externe situé dans l'espace extérieur (B) à l'extérieur de l'automobile dans l'espace intérieur (A) de l'automobile, et le diffuseur d'air (C) du dispositif de climatisation (2) est commandé instantanément pour être activé par la pluralité de modules de détection d'air (1a, 1b) dans un état d'un mécanisme de surveillance pour filtrer et purifier la source de pollution de l'air dans l'espace intérieur (A) de l'automobile à travers la pluralité de composants de filtration et de purification (D), de sorte que la source de pollution de l'air dans l'espace intérieur (A) de l'automobile est filtrée et échangée afin de fournir un air propre,
**caractérisé en ce que**
le dispositif de climatisation (2) comprend en outre un élément de commande d'admission (24), l'entrée d'air externe (213) est utilisée pour introduire l'air externe situé dans l'espace extérieur (B) à l'extérieur de l'automobile, et l'entrée d'air (212) est utilisée pour introduire la source de pollution de l'air dans l'espace intérieur (A) de l'automobile, dans lequel l'élément de commande d'admission (24) est commandé par l'unité de pilotage de commande (22) pour sélectionner l'ouverture de l'entrée d'air externe (213), et l'unité de pilotage de commande (22) reçoit et compare la donnée de détection d'air hors véhicule délivrée par le module de détection d'air hors véhicule (1b) et la donnée de détection d'air dans le véhicule délivrée par le module de détection d'air embarqué (1a) pour déterminer s'il est nécessaire d'ouvrir l'entrée d'air externe (213), afin de commander l'introduction ou la non introduction de l'air externe situé dans l'espace extérieur (B) à l'extérieur de l'automobile ou l'aspiration de la source de pollution de l'air présente dans l'espace intérieur (A) de l'automobile à travers l'entrée d'air (212), et
la pluralité de modules de détection d'air (1a, 1b) comprend au moins un module de détection d'air hors véhicule (1b) et au moins un module de détection d'air embarqué (1a), ledit au moins un module de détection d'air hors véhicule (1b) est placé dans l'entrée d'air externe (213) pour détecter l'air externe situé dans l'espace extérieur (B) à l'extérieur de l'automobile et pour transmettre une donnée de détection d'air hors véhicule, et ledit au moins un module de détection d'air embarqué (1a) est placé dans l'espace intérieur (A) de l'automobile pour détecter la source de pollution de l'air dans l'espace intérieur (A) de l'automobile et pour transmettre au moins une donnée de détection d'air dans le véhicule,
et **en ce que** l'unité de pilotage de commande (22) reçoit et compare la donnée de détection d'air dans le véhicule délivrée par au moins trois modules de détection d'air embarqués (1a) par le calcul d'une intelligence artificielle, afin de localiser la position de la source de pollution de l'air dans l'espace intérieur (A) de l'automobile, et de sélectionner et commander de façon intelligente la sortie d'air voisine de la source de pollution de l'air pour qu'elle expulse de l'air en priorité, et dirige la source de pollution de l'air vers l'entrée d'air (212) voisine de celle-ci, dans lequel l'unité de pilotage de commande (22) du dispositif de climatisation (2) sélectionne et commande le reste des sorties d'air (212) pour expulser l'air par le calcul d'une intelligence artificielle, de sorte qu'un flux d'air est produit pour diriger la source de pollution de l'air vers l'entrée d'air (212) voisine de la source de pollution de l'air pour qu'elle soit aspirée afin d'être filtrée rapidement.

2. Système embarqué de prévention contre la pollution de l'air selon la revendication 1, dans lequel la source de pollution de l'air est au moins un élément choisi dans le groupe comprenant les particules en suspension, le monoxyde de carbone, le dioxyde de carbone, l'ozone, le dioxyde de soufre, le dioxyde d'azote, le plomb, les composés organiques volatils totaux (COVT), le formaldéhyde, les bactéries, les champignons, les virus et une combinaison de ceux-ci.

3. Système embarqué de prévention contre la pollution de l'air selon la revendication 1, dans lequel le composant de filtration et de purification (D) comprend un filtre à air à très haute efficacité (HEPA) (D2), dans lequel l'état du mécanisme de surveillance est activé quand au moins l'une des données de détection d'air de la source de pollution de l'air détectées par la pluralité de modules de détection d'air présents dans l'espace intérieur (A) de l'automobile est supérieure à une valeur de détection sûre.

4. Système embarqué de prévention contre la pollution de l'air selon la revendication 3, dans lequel la valeur de détection sûre est au moins une valeur choisie dans le groupe comprenant un PM2,5 inférieur à 35 µg/m³, une teneur en dioxyde de carbone inférieure à 1000 ppm, des composés organiques volatils totaux (COVT) inférieurs à 0,56 ppm, une teneur en formaldéhyde inférieure à 0,08 ppm, une quantité de bactéries inférieure à 1500 UFC/m³, une quantité de champignons inférieure à 1000 UFC/m³, une teneur en dioxyde de soufre inférieure à 0,075 ppm, une teneur en dioxyde d'azote inférieure à 0,1 ppm, une teneur en monoxyde de carbone inférieure à 9 ppm, une teneur en ozone inférieure à 0,06 ppm, une teneur en plomb inférieure à 0,15 µg/m³ et une combinaison de celles-ci.

5. Système embarqué de prévention contre la pollution de l'air selon la revendication 1, dans lequel l'unité de pilotage de commande (22) reçoit et compare les données de détection d'air dans le véhicule délivrées par au moins trois modules de détection d'air embarqués (1a) par le calcul d'une intelligence artificielle, afin de localiser la position de la source de pollution de l'air dans l'espace intérieur (A) de l'automobile, et de sélectionner et commander de façon intelligente le composant de filtration et de purification (D) placé dans la position de l'entrée d'air (212) voisine de la source de pollution de l'air pour accélérer le transport en vue du filtrage.

6. Système embarqué de prévention contre la pollution de l'air selon la revendication 1, comprenant en outre au moins un dispositif de purification et de filtration (3), et le dispositif de purification et de filtration (3) comprend le diffuseur d'air (C) et le composant de filtration et de purification (D), et le module de détection d'air embarqué (1a) est associé au dispositif de purification et de filtration (3) pour commander l'activation du diffuseur d'air (C), de sorte que la source de pollution de l'air présente dans l'espace intérieur (A) de l'automobile est guidée pour entrer dans le composant de filtration et de purification (D) du dispositif de purification et de filtration (3) pour être filtrée et purifiée.

7. Système embarqué de prévention contre la pollution de l'air selon la revendication 6, dans lequel l'unité de pilotage de commande (22) du dispositif de climatisation (2) reçoit et compare les données de détection d'air dans le véhicule délivrées par au moins trois modules de détection d'air embarqués (1a) par le calcul d'une intelligence artificielle, afin de localiser la position de la source de pollution de l'air dans l'espace intérieur (A) de l'automobile, et de sélectionner et commander de façon intelligente l'activation du dispositif de purification et de filtration (3) voisin de la source de pollution de l'air, de sorte que la source de pollution de l'air peut être aspirée dans le dispositif de purification et de filtration (3) sans diffusion et accélérer le filtrage.

8. Système embarqué de prévention contre la pollution de l'air selon la revendication 6, dans lequel l'unité de pilotage de commande (22) du dispositif de climatisation (2) reçoit et compare les données de détection d'air dans le véhicule délivrées par au moins trois modules de détection d'air embarqués (1a) par le calcul d'une intelligence artificielle, afin de localiser la position de la source de pollution de l'air dans l'espace intérieur (A) de l'automobile, et de sélectionner et commander de façon intelligente le dispositif de purification et de filtration (3) voisin de la source de pollution de l'air pour qu'il soit activé en priorité, dans lequel l'unité de pilotage de commande (22) du dispositif de climatisation (2) sélectionne et commande le reste des dispositifs de purification et de filtration (3) à activer par le calcul d'une intelligence artificielle, de sorte qu'un flux d'air est produit pour diriger la source de pollution de l'air vers le dispositif de purification et de filtration (3) voisin de la source de pollution de l'air pour qu'elle soit aspirée en vue d'une filtration rapide.

9. Système embarqué de prévention contre la pollution de l'air selon la revendication 6, dans lequel ledit au moins un dispositif de purification et de filtration (3) est associé à et incorporé dans une garniture, un siège ou un montant de porte dans l'espace intérieur (A) de l'automobile, et le module de détection d'air embarqué (1a) transmet la donnée de détection d'air dans le véhicule pour qu'elle soit reçue et comparée par l'unité de pilotage de commande (22) du dispositif de climatisation (2) par le calcul d'une intelligence artificielle, et sélectionne et commande de façon intelligente l'activation du dispositif de purification et de filtration (3) voisin de la source de pollution de l'air.

10. Système embarqué de prévention contre la pollution de l'air selon la revendication 6, dans lequel le module de détection d'air embarqué (2) est associé à un dispositif portatif porté sur un corps humain pour détecter la source de pollution de l'air dans l'espace intérieur (A) de l'automobile en temps réel, et pour transmettre la donnée de détection d'air dans le véhicule dans l'espace intérieur (A) de l'automobile pour qu'elle soit reçue et comparée par l'unité de pilotage de commande (22) du dispositif de climatisation (2) par le calcul d'une intelligence artificielle, qui sélectionne et commande de façon intelligente l'activation du dispositif de purification et de filtration (3) voisin de la source de pollution de l'air.

11. Système embarqué de prévention contre la pollution de l'air selon la revendication 6, dans lequel au moins l'un des modules de détection d'air embarqués (1a) est placé sur deux côtés de la pluralité de dispositifs de purification et de filtration (3), de sorte que l'unité de pilotage de commande (22) reçoit et compare les données de détection d'air dans le véhicule délivrées par les modules de détection d'air embarqués (1a) au niveau des positions de la pluralité de dispositifs de purification et de filtration (3), afin d'assurer que la source de pollution de l'air est filtrée par la pluralité de dispositifs de purification et de filtration (3) pour produire de l'air propre introduit dans l'espace intérieur (A) de l'automobile.

12. Système embarqué de prévention contre la pollution de l'air selon la revendication 3, dans lequel la durée de vie du filtre à air HEPA (D2) est calculée d'après le mécanisme de surveillance des données de détection d'air détectées par le module de détection d'air embarqué (1a) et le module de détection d'air hors véhicule (1b) en fonction d'un temps de fonctionnement cumulé du diffuseur d'air (C) dans le dispositif de climatisation (2).

13. Système embarqué de prévention contre la pollution de l'air selon la revendication 1, dans lequel l'unité de pilotage de commande (22) comprend un écran tactile (221) permettant de régler les instructions de commande du dispositif de climatisation (2) en touchant l'écran tactile (221) et d'afficher la donnée de détection d'air.
